(19) Europäisches Patentamt
European Patent Office
Office européen des brevets

(11) **EP 1 232 144 B1**

(12) # FASCICULE DE BREVET EUROPEEN

(45) Date de publication et mention de la délivrance du brevet:
**31.03.2004 Bulletin 2004/14**

(21) Numéro de dépôt: **00985320.1**

(22) Date de dépôt: **24.11.2000**

(51) Int Cl.⁷: **C07D 209/00**

(86) Numéro de dépôt international:
**PCT/FR2000/003278**

(87) Numéro de publication internationale:
**WO 2001/038305 (31.05.2001 Gazette 2001/22)**

(54) **ANTAGONISTES DES RECEPTEURS DE L'IL-8**

IL-8 REZEPTOR ANTAGONISTEN

NOVEL IL-8 RECEPTOR ANTAGONISTS

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **25.11.1999 FR 9914837**

(43) Date de publication de la demande:
**21.08.2002 Bulletin 2002/34**

(73) Titulaire: **FOURNIER INDUSTRIE ET SANTE**
**21300 Chenôve (FR)**

(72) Inventeurs:
- **PAQUET, Jean-Luc**
  **F-21490 Brognon (FR)**
- **BARTH, Martine**
  **F-21380 Asnieres Les Dijon (FR)**
- **PRUNEAU, Didier**
  **F-21370 Pasques (FR)**
- **DODEY, Pierre**
  **F-21121 Fontaine Les Dijon (FR)**

(74) Mandataire: **Hubert, Philippe et al**
**Cabinet Beau de Loménie**
**158, rue de l'Université**
**75340 Paris Cédex 07 (FR)**

(56) Documents cités:
**WO-A-00/51984** **WO-A-96/18393**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne de nouveaux composés inhibant l'action des CXC chimiokines telles que l'IL-8, le Gro, le NAP-2, l'ENA-78 etc. sur leur récepteurs, leur procédé de préparation, ainsi que leur utilisation pour l'obtention de médicaments.

## Art antérieur

L'IL-8 (Interleukine-8) est une protéine de 72 acides aminés appartenant à la superfamille de protéines capables d'attirer les leucocytes, aussi qualifiées de cytokines C-X-C ou C-C cytokines intercrines ou plus récemment de chimiokines (Oppenheim *et al.*, *Annu. Rev Immunol.,* 1991, **9**, 617-648). Différents noms ont été attribués à l'interleukine-8 tels que NAP-1 (neutrophil attractant/activation protein-1), NAF (neutrophil activating factor) et T-cell lymphocyte chemotactic factor. De nombreux membres de la famille des chimiokines ont été décrits comme étant impliqués dans les processus inflammatoires et dans la migration des leucocytes. La famille des chimiokines est composée de deux sous familles distinctes : les alpha- et les béta-chimiokines. Les alpha-chimiokines comme l'IL-8, le NAP-2 (Neutrophil activating peptide-2), le MGSA/Gro, ou Gro-alpha (melanoma growth stimulatory activity), et l'ENA-78, ont toutes des effets sur l'attraction et l'activation des leucocytes et plus particulièrement des neutrophiles. Cette sous-famille inclut aussi le PF-4 (Platelet Factor-4), la béta-thromboglobuline et le CTAPIII, qui eux n'ont pas d'effet sur les neutrophiles.

L'IL-8 a été originellement identifiée par ses capacités à attirer et activer les leucocytes polymorphonucléaires (neutrophiles). Plus récemment, il a été montré que l'expression d'IL-8 était rapidement induite dans différents tissus ou cellules comme les macrophages, les fibroblastes, les cellules endothéliales et épithéliales et même les neutrophiles, en réponse à des cytokines pro-inflammatoires comme l'IL-1 alpha ou béta ou le TNF alpha ou d'autres agents pro-inflammatoires comme le LPS (Van Damme J., *Interleukin-8 and related chemotactic cytokines* ; 1994 ; *The Cytokines Handbook*, 2$^{ème}$ Ed. A.W. Thomson éditeur, Academic Press, London, pp : 185-208). De plus, certaines données de la littérature ont mis en évidence des taux systémiques d'IL-8 élevés dans certaines pathologies inflammatoires impliquant les neutrophiles, suggérant que l'IL-8 et d'autres chimiokines de la même famille, peuvent être des médiateurs fondamentaux de l'activation des neutrophiles (Van Damme, *Interleukin-8 and related chemotactic cytokines* ; 1994 ; *The Cytokines Handbook,* 3$^{ème}$ Ed. A.W. Thomson éditeur, Academic Press, London, pp : 271-311).

Le Gro-alpha, le Gro-béta, le Gro-gamma et le NAP-2 appartiennent à la famille des chimiokines et, comme l'IL-8 , ces protéines elles aussi ont été dénommées par différents termes. Ainsi, les Gro-alpha, béta et gamma ont été appelés respectivement MGSA (Melanoma Growth Stimulatory Activity) a, b et g (Richmond and Thomas, *J. Cell Physiol*., 1986, **129**, 375-384 ; Cheng *et al*., *J. Immunol*., 1992, **148**, 451-456). Toutes ces chimiokines appartiennent au groupe des alpha-chimiokines qui possèdent un motif ELR (Aspartate-Leucine-Arginate) en amont du motif CXC caractéristique de ce sous groupe. Ces chimiokines se lient toutes au récepteur de type 2 ou CXCR2.

Deux récepteurs de l'IL-8 appartenant à la famille des récepteurs à sept domaines trans-membranaires couplés aux protéines G ont été caractérisés et clonés : le récepteur de l'IL-8 de type A (IL-8RA) ou CXCR1 qui lie avec une forte affinité l'IL-8 et le GCP-2, et le récepteur de l'IL-8 de type B (IL-8RB) ou CXCR2 qui a comme ligands spécifiques l'IL-8, le GCP-2, le Gro-alpha, le Gro-béta, le Gro-gamma et le NAP-2 (Ponath, *Exp. Opin. Invest. Drugs, 1998*, **7**, 1-18). Ces deux récepteurs possèdent une homologie de séquence en acides aminés de 77%. De nombreuses publications ont mis en évidence des taux anormalement élevés d'IL-8 dans la polyarthrite rhumatoïde, le choc septique, l'asthme, la mucoviscidose, l'infarctus du myocarde, et le psoriasis (Baggiolini *et al*., *FEBS Lett*., 1992, **307**, 97-101 ; Mille and Krangel., *Crit. Rev. Immunol.,* 1992, **12**, 17-46 ; Oppenheim *et al., Annu. Rev. Immunol.,* 1991, **9**, 617-648 ; Seitz *et al*., *J. Clin. Invest*., 1991, **87**, 463-469 ; Miller *et al., Am. Rev. Resp. Dis.,* 1992, **146**, 427-432 ; Donnelly *et al., Lancet*, 1993, **341**, 643-647). L'IL-8 semble être impliquée dans les phénomènes d'ischémie-reperfusion du poumon (Sekido *et al*., *Nature*, 1993, **365**, 654-657). Un anticorps dirigé contre l'IL-8 ayant la capacité de bloquer la migration *in vitro* des neutrophiles de lapin induite par l'IL-8, prévient les dommages tissulaires résultant d'un processus d'ischémie/reperfusion pulmonaire chez le lapin. L'IL-8 semble jouer un rôle majeur dans les altérations dues à une hypoxie/reperfusion du myocarde (Kukielka *et al*., *J. Clin. Invest*., 1995, **95**, 89-103).

Plus récemment, une autre étude a mis en évidence des effets bénéfiques d'un anticorps neutralisant de l'IL-8 dans un modèle de pleurésie induite par des endotoxines chez le lapin (Broadus *et al*, *J. Immunol.,* 1994, **152**, 2960-2967). L'implication de l'IL-8 dans les inflammations du poumon ainsi que son rôle délétère ont été mis en évidence à l'aide d'anticorps neutralisants de l'IL-8 dans un modèle d'atteinte pulmonaire induite par une instillation d'acide dans les poumons du lapin (Folkesson *et al*., *J. Clin. Invest.,1995,* **96**, 107-116) et dans un modèle de syndrome de détresse respiratoire aiguë induit par des endotoxines (Yokoi *et al*., *Lab. Invest*., 1997, **76**, 375-384). D'autres rapports ont montré des effets bénéfiques similaires avec des anticorps neutralisants de l'IL-8 dans des modèles animaux de dermatose, d'arthrite et de glomérulonéphrite (Akahoshi *et al*., *Lymphokine and Cytokine Res.,* 1994, **13**, 113-116 ; Nishimura *et al*., *J. Leukoc. Biol.,* 1997, **62**, 444-449 ; Wada *et al*., *J. Exp. Med.,* 1994, **180**, 1135-1140). De plus, des

souris déficientes en récepteurs de l'interleukine-8 ont été générées par élimination du gène codant pour le récepteur murin de l'IL-8 homologue au récepteur humain de type 2 (CXCR2) (Cacalano *et al.*, *Science,* 1994, **265**, 682-684). Bien que ces souris soient saines, les caractéristiques de leurs neutrophiles sont modifiées. En effet, leur capacité de migration dans le péritoine est diminuée en réponse à une injection intra-péritonéale de thioglycolate.

Tous ces résultats suggèrent que les chimiokines de la famille de l'IL-8 sont d'importants médiateurs de la migration et de l'activation des neutrophiles et d'autres types cellulaires telles que les cellules endothéliales dans certaines conditions inflammatoires. De plus, les chimiokines de la famille de l'IL-8 ont été décrites comme jouant un rôle important dans la croissance tumorale, la formation de métastases et l'angiogénèse tumorale dans de nombreux types de cancers (Hebert and Baker, *Cancer Invest.*, 1993, **11**, 743-750 ; Richards *et al.*, *Am. J. Surg.*, 1997, **174**, 507-512).

L'étude des propriétés des chimiokines de la famille de l'IL-8 peut laisser supposer que des composés susceptibles d'antagoniser ces chimiokines au niveau de leurs récepteurs, pourraient présenter un potentiel pour atténuer les conséquences de leur action dans certaines pathologies. On connaît ainsi, selon WO 96-18393 des composés dérivés de l'acide 1-benzyl-2-indolecarboxylique qui sont capables de se lier aux récepteurs de l'IL-8 avec un effet inhibiteur. Plus récemment, selon WO 99-06354, des composés dérivés de l'urée ou de la thiourée ont également été présentés comme antagonistes des récepteurs à l'IL-8.

**But de l'invention**

L'invention propose des composés non peptidiques nouveaux qui ont la propriété de se lier au récepteur CXCR2 de l'IL-8 et autres chimiokines de la même famille, en se comportant comme antagonistes de ces récepteurs.

Cette propriété des composés selon l'invention permet d'envisager leur utilisation en tant que principes actifs de médicaments destinés au traitement préventif ou curatif de maladies dans lesquelles les récepteurs de l'IL-8 et chimiokines de la même famille sont impliqués, telles que, par exemple, la polyarthrite rhumatoïde, le psoriasis ou les dermatites atypiques, les maladies associées à une angiogénèse pathologique (comme le cancer), la prolifération des cellules tumorales et la formation de métastases (dans le cas du mélanome par exemple), l'asthme, l'obstruction chronique des poumons, le syndrome de détresse respiratoire aiguë, l'inflammation du colon, la maladie de Crohn, la colite ulcérative, l'ulcère gastrique, le choc septique, le choc endotoxinique, la septicémie à gram-négatif, le syndrome de choc toxique, l'ischémie cérébrale, les phénomènes d'ischémie/reperfusion cardiaques ou rénaux, la glomérulo-néphrite, la thrombose, la maladie d'Alzheimer, les réactions du greffon contre l'hôte ou les rejections d'allogreffes.

**Description**

Selon l'invention, on propose des composés nouveaux de formule :

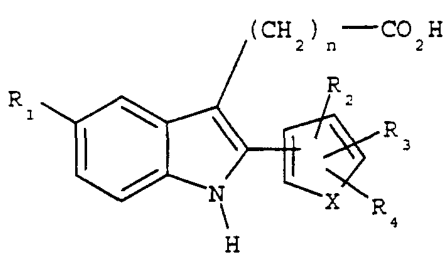

(I)

dans laquelle

X représente une double liaison -C=C- ou un atome de soufre,
$R_1$ représente un halogène, un groupe nitro, un groupe trifluorométhyle ou un groupe alkyle en $C_1$-$C_3$,
$R_2$, $R_3$ et $R_4$ représentent chacun indépendamment un atome d'hydrogène, un halogène, un groupe alkyle en $C_1$-$C_3$, un groupe nitro, un groupe trifluorométhyle ou un groupe cyano, ou $R_2$ et $R_3$ forment ensemble, avec le noyau aromatique auquel ils sont rattachés, un cycle aromatique condensé,
et n est égal à 2 ou 3.

L'invention concerne également en tant que produits nouveaux les esters et les sels d'addition avec une base minérale ou organique des composés de formule I.

Elle concerne également l'utilisation d'un composé de formule I ou de ses sels, pour la préparation d'un

médicament destiné au traitement préventif ou curatif des maladies dépendantes d'une activation des récepteurs de l'IL-8, comme par exemple, la polyarthrite rhumatoïde, le syndrome de détresse respiratoire aiguë, le psoriasis, la maladie de Crohn, et d'une façon plus générale, toute pathologie liée à une infiltration massive de neutrophiles.

## Description détaillée

Comme indiqué précédemment, les composés selon l'invention répondent à la formule I ci-dessus. Selon les définitions des substituants $R_1$ à $R_4$, on comprend par halogène les atomes de fluor, de chlore et de brome, les atomes de fluor et de chlore étant préférés. Par groupe alkyle en $C_1$-$C_3$, il faut comprendre les groupes méthyle, éthyle, propyle, 1-méthyléthyle et cyclopropyle.

Parmi les composés de l'invention, on préfère les composés de la formule (Ia) suivante :

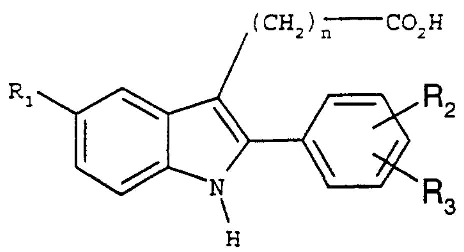
(Ia)

dans laquelle $R_1$ représente un halogène, un groupe nitro, un groupe trifluorométhyle ou un groupe alkyle en $C_1$-$C_3$, $R_2$ et $R_3$ représentent chacun indépendamment un atome d'hydrogène, un halogène, un groupe alkyle en $C_1$-$C_3$, ou forment ensemble, avec le noyau phényle auquel ils sont attachés, un cycle aromatique condensé,
et n est égal à 2 ou 3,
ainsi que leurs esters et sels d'addition avec une base minérale ou organique.

Parmi les composés selon l'invention, on préfère plus particulièrement les composés de formule I dans lesquels X est une double liaison -C=C-, $R_1$ est un atome de chlore, $R_2$ et/ou $R_3$ représentent chacun un atome de chlore ou de fluor, ou un groupe méthyle, de préférence en position méta et/ou para du noyau phényle, et $R_4$ est l'atome d'hydrogène.

Par cycle aromatique condensé, on comprend un cycle formant, avec le noyau aromatique supportant les substituants $R_2$, $R_3$, $R_4$, un groupe à 2 cycles aromatiques condensés tel que par exemple un groupe 2-naphtyle ou 1-naphtyle, le groupe 2-naphtyle étant préféré.

Les composés de formule I, qui sont des acides, peuvent être estérifiés par des alcools organiques, notamment des alcools aliphatiques en $C_2$-$C_3$ tels que l'éthanol ou l'isopropanol (ou 1-méthyléthanol). Parmi ces esters, on préfère les esters éthyliques.

Les composés de formule I peuvent être salifiés avec une base minérale ou organique. Par base minérale, on comprend les hydroxydes des métaux alcalins tels la soude, la potasse, la lithine ou alcalino-terreux tels que la chaux. Par base organique, on entend les amines primaires, secondaires ou tertiaires, les aminoalcools, certains hétérocycles azotés non toxiques, ainsi que les acides aminés basiques. Parmi les sels, on préfère les sels de sodium ou de potassium, et les sels de lysine, d'arginine ou de 2-amino-2-méthyl-1,3-propanediol.

Les composés de formule I peuvent être notamment préparés par un procédé comprenant les étapes consistant à :

a) faire réagir selon une réaction de type Friedel-Crafts un anhydride cyclique de diacide de formule :

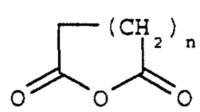
(II)

dans laquelle n est égal à 2 ou 3
avec un dérivé aromatique de formule :

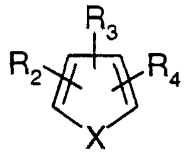

(III)

dans laquelle X représente une liaison -C=C- ou un atome de soufre, $R_2$, $R_3$ et $R_4$ représentent chacun indépendamment un atome d'hydrogène, un halogène, un groupe alkyle en $C_1$-$C_3$ ou $R_2$ et $R_3$ forment ensemble avec le noyau aromatique auquel ils sont rattachés un cycle aromatique condensé,
dans un solvant anhydre comme par exemple le dichlorométhane, en présence d'un acide de Lewis comme par exemple le chlorure d'aluminium, à une température comprise entre -10 et +50°C, pour obtenir un composé de formule :

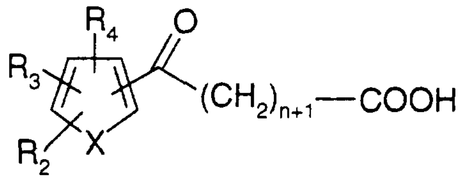

(IV)

dans laquelle X, $R_2$, $R_3$, $R_4$ et n conservent la même signification que ci-dessus,
b) estérifier le composé de formule IV ci-dessus par exemple avec un alcool aliphatique de formule ROH (R= Me ou Et), dans des conditions classiques connues de l'homme de l'art pour obtenir un ester de formule :

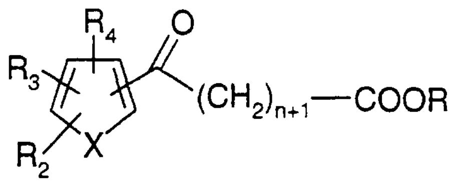

(V)

dans laquelle X, R, $R_2$, $R_3$, $R_4$ et n conservent la même signification que précédemment,
c) faire réagir, selon la réaction de Fischer, le composé de formule V avec une phénylhydrazine de formule :

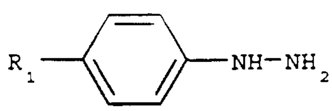

(VI)

dans laquelle $R_1$ représente un halogène, un groupe trifluorométhyle, ou un groupe alkyle en $C_1$-$C_3$,
en présence de chlorure de zinc, dans un solvant comme par exemple l'acide acétique, à une température de l'ordre de 20 à 80 °C, pour obtenir le dérivé de l'indole de formule :

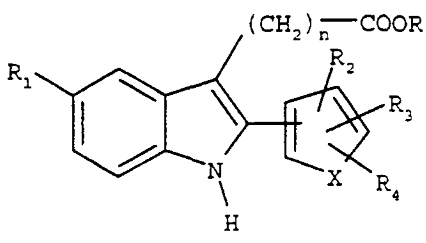

(Ie)

dans laquelle X, R, $R_1$, $R_2$, $R_3$, $R_4$ et n conservent la même signification que précédemment,

d) hydrolyser la fonction ester du composé de formule Ie obtenue précédemment, selon une réaction connue de l'homme de l'art comme par exemple par réaction avec une solution hydro-alcoolique d'hydroxyde de sodium, pour obtenir le dérivé acide correspondant de formule :

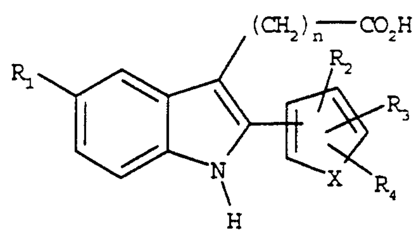

(I)

dans laquelle X, $R_1$, $R_2$, $R_3$, $R_4$ et n conservent la même signification que ci-dessus,

e) si nécessaire, préparer un sel de l'acide de formule I par réaction du composé de formule I avec un composé minéral ou organique basique.

Les composés de formule (I) dans laquelle $R_1$ est un groupe nitro peuvent être obtenus par nitration des composés correspondants dans lesquels $R_1$ est l'hydrogène selon des procédés classiques bien connus de l'homme du métier.

Les composés de formule (Ia) peuvent être préparés par le procédé ci-dessus dans lequel on utilise un dérivé aromatique de formule (III) dans laquelle X représente une double liaison -C=C-, $R_4$ est un atome d'hydrogène et $R_2$ et $R_3$ représentent chacun indépendamment un atome d'hydrogène, un halogène, un groupe alkyle en $C_1$-$C_3$, ou forment ensemble, avec le noyau phényle auquel ils sont attachés, un cycle aromatique condensé.

Les composés de formule (V) précités peuvent également être obtenus directement par réaction de type Friedel-Crafts entre un chlorure d'acide de formule :

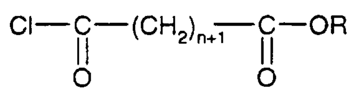

et un dérivé aromatique de formule (III) tel que défini précédemment.

Selon une variante du procédé d'obtention des composés de formule I, on effectue les réactions consistant à :

a) introduire un atome de brome en position 2 d'un dérivé de l'indole de formule VII :

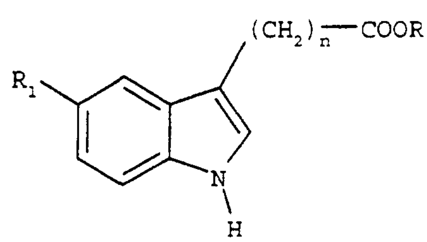

VII

dans laquelle R représente un groupe méthyle, $R_1$ représente un halogène, un groupe trifluoromethyle, un groupe nitro ou un groupe alkyle en $C_1$-$C_3$ et n représente 2 ou 3, notamment par action du N-bromosuccinimide, dans un solvant tel que le tétrachlorure de carbone, pour obtenir le composé de formule :

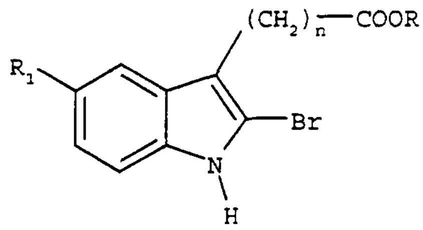

VIII

dans laquelle n, R et $R_1$ restent inchangés,

b) introduire un groupe aromatique substitué ou non substitué, en substitution de l'atome de brome en position 2 du composé de formule VIII, notamment par action d'un acide boronique de formule :

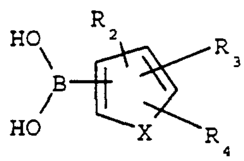

IX

dans laquelle $R_2$, $R_3$ et $R_4$ représentent chacun indépendamment un atome d'hydrogène, un groupe alkyle en $C_1$-$C_3$, un atome de chlore, un atome de fluor, un groupe trifluorométhyle ou un groupe cyano, et X représente une double liaison -C=C- ou un atome de soufre,

dans un solvant et en présence d'un catalyseur tel que le tétrakis(triphénylphosphine)palladium, pour obtenir un composé de formule :

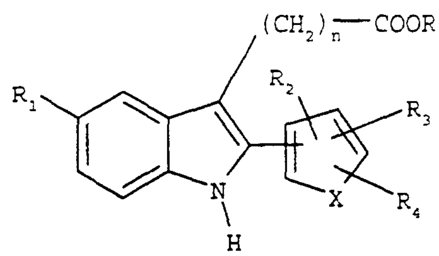

Ie

dans laquelle X, R, $R_1$, $R_2$, $R_3$, $R_4$ et n conservent la même signification que précédemment,

c) hydrolyser la fonction ester du composé de formule Ie, suivant une procédure analogue à celle préconisée au stade d) du procédé décrit précédemment, pour obtenir le composé de formule I :

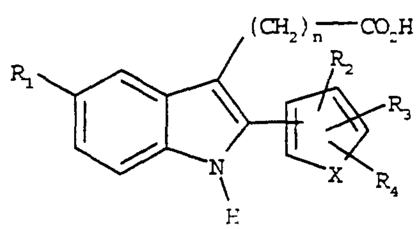

I

dans laquelle X, $R_1$, $R_2$, $R_3$, $R_4$ et n conservent la même signification que précédemment.

Selon une variante de ce procédé, l'étape b) consiste à faire réagir le composé de formule VIII avec un dérivé de l'étain ayant un cycle aromatique nitré, tel que par exemple le triméthyl(4-nitrophényl)étain, selon des procédés classiques bien connus de l'homme du métier pour former les composés de formule (I) dans laquelle $R_2$, $R_3$ ou $R_4$ est un groupe

nitro.

**PREPARATION I**

**Acide 4-fluoro-ε-oxo-benzènehexanoïque, méthyl ester**

On prépare une suspension de 2,59 g ($19,4.10^{-3}$ mole) de chlorure d'aluminium dans 4 ml de dichlorométhane. On refroidit à - 5 °C et on ajoute progressivement un mélange de 0,97 ml ($10,3.10^{-3}$ mole) de fluorobenzène et 1,31 ml ($8,4.10^{-3}$ mole) de l'ester méthylique de l'acide 6-chloro-6-oxo-hexanoïque dans 3 ml de dichlorométhane en maintenant la température entre - 4 et - 7 °C. On laisse ensuite remonter la température jusqu'à 20 °C et, après 15 heures, on hydrolyse sur de l'eau glacée acidifiée. Le mélange est extrait par du dichlorométhane et la phase organique obtenue est lavée à l'eau, séchée sur sulfate de magnésium et concentrée sous pression réduite. On récupère ainsi 2 g de produit brut que l'on purifie par chromatographie sur gel de silice en éluant avec un mélange éther de pétrole/acétate d'éthyle (96/4). On obtient ainsi 1,26 g du produit attendu sous forme d'une poudre blanche. (Rendement = 63 %)
F = 58-59 °C

**PREPARATION II**

**Acide 3,4-dichloro-ε-oxo-benzènehexanoïque, méthyl ester**

En opérant de façon analogue à la préparation I, au départ de 1,2-dichlorobenzène, on obtient le produit attendu sous forme d'un solide ocre avec un rendement de 79 %.
F = 41-44 °C

**PREPARATION III**

**Acide ε-oxo-2-naphtalènehexanoïque, méthyl ester**

En opérant de façon analogue à la préparation I, au départ de naphtalène, on obtient le produit attendu sous forme d'un solide beige avec un rendement de 53 %.
F = 58-60 °C

**PREPARATION IV**

**Acide 4-fluoro-δ-oxo-benzènepentanoïque**

On prépare une suspension de 22,32 g (0,167 mole) de chlorure d'aluminium dans 35 ml de dichlorométhane. On refroidit à 0 °C et on ajoute doucement un mélange de 8,3 g (0,0728 mole) d'anhydride glutarique (dihydro-2-*H*-pyran-2,6(3*H*)-dione), 8,4 ml (0,0895 mole) de fluorobenzène dans 20 ml de dichlorométhane. On laisse le mélange sous agitation pendant 15 heures à température ambiante puis on hydrolyse sur de l'eau glacée acidifiée. Le produit précipité est filtré et lavé à l'eau, puis séché sous pression réduite. Le produit brut est ensuite recristallisé dans 90 ml d'acétate d'éthyle. On obtient ainsi 8,8 g du produit attendu sous forme de cristaux beige (Rendement = 57,5 %)
F = 134-136 °C

**PREPARATION V**

**Acide 4-chloro-δ-oxo-benzènepentanoïque**

En opérant de façon analogue à la préparation IV, au départ de chlorobenzène, on obtient le produit attendu sous forme d'un solide brun avec un rendement de 39 %.
F = 108-110 °C

**PREPARATION VI**

**Acide 4-méthyl-δ-oxo-benzènepentanoïque**

En opérant de façon analogue à la préparation IV, au départ de toluène, on obtient le produit attendu sous forme d'un solide beige avec un rendement de 34 %.

F = 131-133 °C

**PREPARATION VII**

**Acide 4-fluoro-δ-oxo-benzènepentanoïque, éthyl ester**

On prépare une suspension de 8.76 g ($41,7.10^{-3}$ mole) d'acide obtenu selon la préparation IV dans 80 ml d'éthanol et on ajoute 1,33 ml d'acide sulfurique pur. Le mélange est porté à reflux sous agitation pendant 5 heures. Le milieu réactionnel est ensuite concentré sous pression réduite puis repris dans l'éther éthylique. Cette phase organique est lavée à l'eau, puis avec une solution de soude diluée, puis à nouveau à l'eau. Après séchage sur sulfate de magnésium, le solvant est chassé sous pression réduite et on obtient 9,65g du produit attendu sous forme d'un solide orange pâle (Rendement = 97 %)
F = 46-47 °C

**PREPARATION VIII**

**Acide 4-chloro-δ-oxo-benzènepentanoïque, éthyl ester**

En opérant de façon analogue à la préparation VII, au départ d'acide obtenu selon la préparation V, on obtient le produit attendu sous forme d'un solide brun avec un rendement de 87 %.
F = 45-48 °C

**PREPARATION IX**

**Acide 4-méthyl-δ-oxo-benzènepentanoïque, éthyl ester**

En opérant de façon analogue à la préparation VII, au départ d'acide obtenu selon la préparation VI, on obtient le produit attendu sous forme d'un solide brun avec un rendement de 65 %.
F = 36-38 °C

**Exemple 1**

**Acide 5-bromo-2-phényl-1*H*-indole-3-butanoïque**

a) acide ε-[(E)-2-(4-bromophényl)hydrazono]-benzènehexanoïque, éthyl ester

On prépare un mélange de 1,91 g ($8,55.10^{-3}$ mole) de chlorhydrate de 4-bromophénylhydrazine et 0,73 g ($8,9.10^{-3}$ mole) d'acétate de sodium dans 17 ml d'eau et on ajoute 2,05 ml ($35,8.10^{-3}$ mole) d'acide acétique. On porte ensuite ce mélange sous agitation à 70 °C puis on ajoute doucement 2,0 g ($8,54.10^{-3}$ mole) d'ester éthylique de l'acide ε-oxo-benzènehexanoïque en suspension dans 27 ml d'eau. Le mélange réactionnel est maintenu sous agitation à 70-80 °C pendant 45 mn, puis à température ambiante pendant 12 heures, puis extrait par 2 fois 50 ml d'acétate d'éthyle. La phase organique est lavée à l'eau, séchée sur sulfate de magnésium et concentrée sous pression réduite. On obtient ainsi 3,38 g du composé attendu utilisé directement dans l'étape suivante.

b) acide 5-bromo-2-phényl-1*H*-indole-3-butanoïque

On prépare un mélange de 0,6 g de chlorure de zinc et 1,78 g du composé obtenu au stade a) ci-dessus, dans 4,4 ml d'acide acétique. Ce milieu réactionnel est porté à 75-85 °C pendant 3 heures puis refroidi à température ambiante (env. 20 °C). On ajoute 20 ml d'eau puis 40 ml d'acétate d'éthyle. La phase aqueuse est séparée et extraite à nouveau par 30 ml d'acétate d'éthyle et les phases organiques rassemblées sont lavées à l'eau, séchées sur sulfate de magnésium et concentrées sous pression réduite. On obtient ainsi 1,74 g de produit huileux (ester de l'acide attendu) que l'on reprend avec 10 ml d'une solution d'hydroxyde de sodium à 10 % dans l'éthanol. Ce mélange est chauffé à reflux pendant 30 mn, puis refroidi à 20 °C. On ajoute 40 ml d'eau et on chasse l'éthanol sous pression réduite à 40-45 °C. La phase aqueuse basique résiduelle est lavée par 10 ml d'acétate d'éthyle, puis acidifiée jusqu'à pH 1 par une solution d'acide chlorhydrique dilué, et extraite avec deux fois 75 ml d'acétate d'éthyle. Cette phase organique est lavée à l'eau, séchée et concentrée sous pression réduite. On obtient ainsi 1,8 g de produit brut que l'on purifie par chromatographie sur gel de silice en éluant par un mélange hexane/acétate d'éthyle (7/3) ; cette purification conduit à 600 mg de l'acide attendu sous forme d'un solide rose (rendement = 38 %).

F = 155-157 °C

**Exemple 2**

**Acide 5-chloro-2-phényl-1*H*-indole-3-propanoïque**

En opérant de façon analogue à l'exemple 1, au départ de 4-chlorophénylhydrazine et de l'ester éthylique de l'acide δ-oxo-benzènepentanoïque, on obtient l'acide attendu sous forme d'un solide blanc avec un rendement de 11 %.
F=167°C

**Exemple 3**

**Acide 5-chloro-2-phényl-1*H*-indole-3-butanoïque**

En opérant de façon analogue à l'exemple 1, au départ de 4-chlorophénylhydrazine, on obtient l'acide attendu sous forme d'un solide blanc avec un rendement de 19 %.
F = 169-170 °C

**Exemple 4**

**Acide 5-fluoro-2-phényl-1*H*-indole-3-butanoïque**

En opérant de façon analogue à l'exemple 1, au départ de 4-fluorophénylhydrazine, on obtient l'acide attendu sous forme d'un solide beige avec un rendement de 15 %.
F = 156-157 °C

**Exemple 5**

**Acide 5-(trifluorométhyl)-2-phényl-1*H*-indole-3-butanoïque**

En opérant de façon analogue à l'exemple 1, au départ de 4-(trifluorométhyl)phénylhydrazine, on obtient l'acide attendu sous forme d'un solide orange avec un rendement de 12 %.
F = 144 °C

**PREPARATION X**

**Acide 5-chloro-2-(4-fluorophényl)-1*H*-indole-3-propanoïque, éthyl ester**

On prépare un mélange de 13,9 g ($40,3.10^{-3}$ mole) de l'ester obtenu selon la préparation VII, 10,8 g ($60,4.10^{-3}$ mole) de chlorhydrate de 4-chlorophénylhydrazine, 5,5 g ($40,3.10^{-3}$ mole) de chlorure de zinc dans 80 ml d'acide acétique. Ce mélange est porté à 65-70 °C et maintenu sous agitation à cette température pendant 18 heures. Après refroidissement, le milieu réactionnel est filtré et le filtrat est hydrolysé sur de l'eau froide. Le composé organique précipité est extrait par 2 fois 150 ml d'acétate d'éthyle. La phase organique obtenue est lavée à l'eau, séchée sur sulfate de magnésium et concentrée sous pression réduite. On obtient 15 g de produit brut que l'on recristallise dans un mélange éther diéthylique/éther de pétrole. On obtient ainsi 8,2 g de produit, que l'on purifie à nouveau par chromatographie sur gel de silice en éluant avec un mélange toluène/acétate d'éthyle (95/5), pour donner 6,93 g du produit attendu sous forme d'un solide jaune (rendement = 50 %).
F = 104-105 °C

**Exemple 6**

**Acide 5-chloro-2-(4-fluorophényl)-1*H*-indole-3-propanoïque**

On prépare un mélange de 500 mg ($1,45.10^{-3}$ mole) de l'ester obtenu selon la préparation X, dans 10 ml de dioxane. On ajoute 3 ml d'une solution de soude 3N et on porte le milieu réactionnel à reflux pendant 2 heures. Le solvant est ensuite éliminé sous pression réduite et le résidu est repris dans 30 ml d'eau. La solution obtenue est acidifiée par de l'acide chlorhydrique N. Le précipité formé est extrait par 2 fois 50 ml d'acétate d'éthyle. Les phases organiques rassemblées sont lavées à l'eau, séchées sur sulfate de magnésium puis concentrées sous pression réduite. Le produit brut est recristallisé dans un mélange acétate d'éthyle/éther de pétrole pour donner 200 mg de l'acide attendu sous forme d'une poudre beige (rendement = 43,5 %).
F = 153-154 °C

## PREPARATION XI

### Acide 5-chloro-2-(4-chlorophényl)-1*H*-indole-3-propanoïque, éthyl ester

En opérant de façon analogue à la préparation X, au départ du composé obtenu selon la préparation VIII, on obtient l'ester attendu sous forme d'une huile brune avec un rendement de 35 %.
RMN $^1H$ ($CDCl_3$, 300MHz) $\delta$ : 8,09 (s large, 1H) ; 7,90 (d, J=8,4Hz, 1H) ; 7,58 (d, J=2,2Hz, 1H) ; 7,47 (m, 2H) ; 7,43 (d, J=8,4Hz ; 1H) ; 7,28 (d, J= 8,4Hz, 1H) ; 7,16 (dd, J= 8,4Hz, J=2,2Hz, 1H) ; 4,16 (q, J= 7Hz, 2H) ; 3,18 (t, J=8Hz, 2H) ; 2,65 (t, J=8Hz, 2H) ; 1,23 (t, J=7Hz, 3H).

## Exemple 7

### Acide 5-chloro-2-(4-chlorophényl)-1*H*-indole-3-propanoïque

En opérant de façon analogue à l'exemple 6, au départ du composé obtenu selon la préparation XI, on obtient l'acide attendu sous forme d'un solide beige avec un rendement de 40 %.
F = 187-190 °C

## PREPARATION XII

### Acide 5-chloro-2-(4-méthylphényl)-1*H*-indole-3-propanoïque, éthyl ester

En opérant de façon analogue à la préparation X, au départ du composé obtenu selon la préparation IX, on obtient l'ester attendu sous forme d'une pâte brune avec un rendement de 97 %.
RMN $^1H$ ($CDCl_3$, 300MHz) $\delta$ : 8,1 (s large, 1H) ; 7,58 (d, J=1,8Hz, 1H) ; 7,42 (dt, J=8,1Hz, J=1,8Hz, 2H) ; 7,29 (d, J=8Hz, 2H) ; 7,25 (d, J=8,8Hz , 1H) ; 7,14 (dd, J= 8,8Hz, J=1,8Hz, 1H) ; 4,20 (q, J= 7Hz, 2H) ; 3,20 (t, J= 8Hz, 2H) ; 2,70 (t, J=8Hz, 2H) ; 1,23 (t, J=7Hz, 3H).

## Exemple 8

### Acide 5-chloro-2-(4-méthylphényl)-1*H*-indole-3-propanoïque

En opérant de façon analogue à l'exemple 6, au départ du composé obtenu selon la préparation XII, on obtient l'acide attendu sous forme d'un solide beige avec un rendement de 40 %.
F = 177-178 °C

## PREPARATION XIII

### Acide 5-chloro-2-(4-chlorophényl)-1*H*-indole-3-butanoïque, méthyl ester

En opérant de façon analogue à la préparation X, au départ de l'ester méthylique de l'acide 4-chloro-ε-oxo-benzènehexanoïque, on obtient le produit attendu sous forme d'un solide marron clair avec un rendement de 90 %.
F = 47-48 °C

## Exemple 9

### Acide 5-chloro-2-(4-chlorophényl)-1*H*-indole-3-butanoïque

En opérant de façon analogue à l'exemple 6, au départ du produit obtenu selon la préparation XIII, on obtient l'acide attendu sous forme d'un solide beige avec un rendement de 20 %.
F = 194-197 °C

**PREPARATION XIV**

**Acide 5-chloro-2-(3,4-dichlorophényl)-1*H*-indole-3-butanoïque, méthyl ester**

En opérant de façon analogue à la préparation X, au départ du composé obtenu selon la préparation II, on obtient l'ester attendu sous forme d'un solide rose avec un rendement de 26 %.
F = 285 °C (décomposition)

**Exemple 10**

**Acide 5-chloro-2-(3,4-dichlorophényl)-1*H*-indole-3-butanoïque**

En opérant de façon analogue à l'exemple 6, au départ du composé obtenu selon la préparation XIV, on obtient l'acide attendu sous forme d'un solide brun avec un rendement de 39 %.
F = 187-188 °C

**PREPARATION XV**

**Acide 5-méthyl-2-phényl-1*H*-indole-3-butanoïque, éthyl ester**

En opérant de façon analogue à la préparation X, au départ de l'ester éthylique de l'acide ε-oxo-benzènehexanoïque et de chlorhydrate de 4-méthylphénylhydrazine, on obtient l'ester attendu sous forme d'un solide brun avec un rendement de 58 %.
F = 96-98 °C

**Exemple 11**

**Acide 5-méthyl-2-phényl-1*H*-indole-3-butanoïque**

En opérant de façon analogue à l'exemple 6, au départ du composé obtenu à partir de la préparation XV, on obtient l'acide attendu sous forme d'un solide brun avec un rendement de 93 %.
F = 150-152 °C

**PREPARATION XVI**

**Acide 5-chloro-2-(4-fluorophényl)-1*H*-indole-3-butanoïque, méthyl ester**

En opérant de façon analogue à la préparation X, au départ du composé obtenu selon la préparation I, on obtient le produit attendu sous forme d'une huile brune avec un rendement de 86 %.
RMN $^1$H (DMSO, 300MHz) δ : 11,4 (s large, 1H) ; 7,66 (d, J=8Hz, 1H) ; 7,64 (d, J=8Hz, 1H) ; 7,63 (d, J=2,4Hz, 1H) ; 7,38 (d, J=8,8Hz, 1H) ; 7,35 (d, J= 8Hz, 1H) ; 7,33 (d, J=8Hz, 1H) ; 7,09 (dd, J= 8,8Hz, J=2,4Hz, 1H) ; 3,55 (s, 3H) ; 2,8 (t, J=7,3Hz, 2H) ; 2.37 (t, J=7,3Hz, 2H) ; 1,85 (quin, J=7,3Hz, 2H).

**Exemple 12**

**Acide 5-chloro-2-(4-fluorophényl)-1*H*-indole-3-butanoïque**

En opérant de façon analogue à l'exemple 6, au départ du composé obtenu selon la préparation XVI, on obtient le produit attendu sous forme d'un solide brun avec un rendement de 53 %.
F = 190-192 °C

**PREPARATION XVII**

**Acide 5-chloro-2-(2-naphtyl)-1*H*-indole-3-butanoïque, méthyl ester**

En opérant de façon analogue à la préparation X, au départ du composé obtenu selon la préparation III, on obtient le produit attendu sous forme d'une pâte orange avec un rendement de 65 %.
RMN$^1$H ($CDCl_3$, 300MHz) δ : 8,2 (s large, 1H) ; 8,01 (s, 1H) ; 7,91 (m, 3H) ; 7,68 (d, J=8,1Hz, 1H) ; 7,62 (s, 1H) ; 7,53

(m, 2H) ; 7,31 (dd, J=8,8Hz, J=2,2Hz, 1H) ; 7,16 (dt, J=6,6Hz, J=2Hz, 1H) ; 3,55 (s, 3H) ; 2,98 (t, J=7,3Hz, 2H) ; 2,37 (t, J=7,3Hz, 2H) ; 2,05 (quin, J=7,3Hz, 2H).

**Exemple 13**

**Acide 5-chloro-2-(2-naphtyl)-1*H*-indole-3-butanoïque**

En opérant de façon analogue à l'exemple 6, au départ du composé obtenu selon la préparation XVII, on obtient le produit attendu sous forme d'un solide brun avec un rendement de 79 %.
F = 180-185 °C

**Exemple 14**

**Acide 2-phényl-5-nitro-1*H*-indole-3-butanoïque**

On ajoute, sous agitation et à 0-5°C, une solution de 1,19 g de nitrate de sodium ($4.10^{-3}$ mole) dans 50 ml d'acide sulfurique concentré à une solution de 3,67 g ($13,15.10^{-2}$ mole) d'acide 2-phényl-1*H*-indole-3-butanoïque dans 200 ml d'acide sulfurique concentré. On maintient l'agitation à 5 °C, pendant 20 mn puis on verse le milieu réactionnel sur un mélange d'eau et de glace. Le précipité jaune formé est filtré et lavé sur le filtre à l'eau et avec un peu d'éther de pétrole. Le produit est ensuite séché sous pression réduite et purifié par chromatographie sur gel de silice en éluant à l'aide d'un mélange hexane/acétate d'éthyle (1/1). On obtient ainsi 1,3 g du produit attendu sous forme d'un solide jaune avec un rendement de 30 %.
F = 145 °C

**Exemple 15**

**Acide 5-chloro-2-(4-fluorophényl)-1*H*-indole-3-butanoïque, sel de sodium**

On prépare une suspension de 1 g ($3,15.10^{-3}$ mole) d'acide obtenu selon l'exemple 12 dans 100 ml d'eau et on ajoute 3,15 ml d'une solution N d'hydroxyde de sodium. Le mélange est agité pendant 30 mn puis filtré sur un filtre 0,45 µm. Le filtrat est lyophilisé et on obtient ainsi 1,05 g du produit attendu sous forme d'un solide fin blanc (rendement = 98 %).
F = 160-162 °C

**PREPARATION XVIII**

**Acide 3,4-dichloro-δ-oxo-benzènepentanoïque, éthyl ester**

En opérant de façon analogue à la préparation VII, au départ de l'acide 3,4-dichloro-δ-oxo-benzènepentanoïque, on obtient le produit attendu sous forme d'une huile marron (rendement = 47 %).
RMN (300MHz, $CDCl_3$) δ : 8.05 (d, J = 1.5 Hz, 1H) ; 7.80 (dd, J = 1.5 Hz, J = 8.1 Hz, 1H) ; 7.56 (d, J = 8.1 Hz, 1H) ; 4.13 (q, J = 7.4 Hz, 2H) ; 3.02 (t, J = 6.6 Hz, 2H) ; 2.42 (t, J = 6.6 Hz, 2H) ; 2.05 (quint, J = 6.6 Hz, 2H) ; 1.25 (t, J = 7.4 Hz, 3H).

**PREPARATION XIX**

**Acide 5-chloro-2-(3,4-dichlorophényl)-1*H*-indole-3-propanoïque, éthyl ester**

En opérant de façon analogue à la préparation X, au départ du composé obtenu selon la préparation XVIII, on obtient le produit attendu sous forme d'une pâte orange (rendement = 55 %).
RMN (300MHz, $CDCl_3$) δ : 8.1 (s, 1H) ; 7.64 (d, J = 1.5 Hz, 1H) ; 7.58 (d, J = 2.2 Hz, 1H) ; 7.55 (d, J = 8.8 Hz, 1H) ; 7.39 (dd, J = 2.2 Hz, J = 8.8 Hz, 1H) ; 7.28 (d, J = 8.8 Hz, 1H) ; 7.17 (dd, J = 1.4 Hz, J = 8.8 Hz, 1H) ; 4.12 (q, J = 8.1 Hz, 2H) ; 3.18 (m, 2H) ; 2.64 (m, 2H) ; 1.25 (t, J = 8.1 Hz, 3H).

**Exemple 16**

**Acide 5-chloro-2-(3,4-dichlorophényl)-1*H*-indole-3-propanoïque**

En opérant de façon analogue à l'exemple 6, au départ du composé obtenu selon la préparation XIX, on obtient le produit attendu sous forme d'un solide blanc cassé (rendement = 36 %).
F = 150-152 °C

**PREPARATION XX**

**Acide 5-chloro-2-(4-bromophényl)-1*H*-indole-3-butanoïque, méthyl ester**

En opérant de façon analogue à la préparation X, au départ de l'ester méthylique de l'acide 4-bromo-ε-oxo-benzènehexanoïque, on obtient le produit attendu sous forme d'un solide orangé (rendement = 89 %).
F = 124-126 °C

**Exemple 17**

**Acide 5-chloro-2-(4-bromophényl)-1*H*-indole-3-butanoïque**

En opérant de façon analogue à l'exemple 6, au départ du composé obtenu selon la préparation XX, on obtient le produit attendu sous forme d'un solide blanc (rendement = 93 %).
F = 194-195 °C

**PREPARATION XXI**

**Acide 5-chloro-2-(4-cyanophényl)-1*H*-indole-3-butanoïque, méthyl ester**

On prépare un mélange de 480 mg ($1,18.10^{-3}$ mole) de l'ester obtenu selon la préparation XX, 870 mg ($9,7.10^{-3}$ mole) de cyanure cuivreux et 2 ml de N-méthyl-2-pyrrolidone que l'on chauffe à reflux pendant 3 heures. Le milieu réactionnel est ensuite refroidi et on ajoute 10 ml d'eau. Le mélange est maintenu sous agitation à température ambiante pendant 15 minutes puis on ajoute 8 ml d'éthylènediamine. Le mélange est ensuite extrait trois fois avec du toluène et les phases organiques rassemblées sont séchées et concentrées sous pression réduite. Le résidu d'évaporation est purifié par chromatographie sur gel de silice en éluant à l'aide d'un mélange hexane/acétate d'éthyle (80/20 ; v/v). On obtient ainsi 220 mg du produit attendu sous forme d'un fin solide blanc (rendement = 53 %).
F = 182-185 °C

**Exemple 18**

**Acide 5-chloro-2-(4-cyanophényl)-1*H*-indole-3-butanoïque**

En opérant de façon analogue à l'exemple 6, au départ du composé obtenu selon la préparation XXI, on obtient le produit attendu sous forme d'un solide jaune pâle (rendement = 22 %).
F = 214-215 °C

**PREPARATION XXII**

**Acide 3,4-difluoro-ε-oxo-benzènehexanoïque, méthyl ester**

En opérant de façon analogue à la préparation I, au départ de 1,2-difluorobenzène, on obtient le produit attendu sous forme d'un solide jaune (rendement = 46 %).
F = 41-43 °C

**PREPARATION XXIII**

**Acide 5-chloro-2-(3,4-difluorophényl)-1*H*-indole-3-butanoïque, méthyl ester**

En opérant de façon analogue à la préparation X, au départ du composé obtenu selon la préparation XXII,

on obtient le produit attendu sous forme d'un solide blanc (rendement = 70 %).
F = 127-128 °C

**Exemple 19**

**Acide 5-chloro-2-(3,4-difluorophényl)-1*H*-indole-3-butanoïque**

En opérant de façon analogue à l'exemple 6, au départ de l'ester obtenu selon la préparation XXIII, on obtient le produit attendu sous forme d'un solide blanc (rendement = 86 %).
F = 185-186 °C

**PREPARATION XXIV**

**Acide 2-bromo-5-chloro-1*H*-indole-3-butanoïque, méthyl ester**

On prépare une solution de 2,25 g ($8,94.10^{-3}$ mole) de l'ester méthylique de l'acide 5-chloro-1*H*-indole-3-butanoïque dans 85 ml de tétrachlorure de carbone et on ajoute 1,75 g ($9,83.10^{-3}$ mole) de N-bromosuccinimide. Le mélange réactionnel est porté à reflux sous agitation pendant 1 heure puis refroidi à température ambiante et filtré. Le solide est lavé au tétrachlorure de carbone et les filtrats sont concentrés sous pression réduite. Le résidu d'évaporation est purifié par chromatographie sur gel de silice en éluant à l'aide d'un mélange éther de pétrole/acétate d'éthyle (9/1 ; v/v). On obtient ainsi 2,11 g du produit attendu sous forme d'un solide beige (rendement = 71 %).
F = 98 °C

**PREPARATION XXV**

**Acide 5-chloro-2-(4-chloro-3-fluorophényl)-1*H*-indole-3-butanoïque, méthyl ester**

On prépare une solution de 0,4 g ($1,21.10^{-3}$ mole) du composé obtenu selon la préparation XXIV et de 0,32 g ($1,83.10^{-3}$ mole) d'acide 4-chloro-3-fluorophénylboronique dans 14 ml d'éthanol et 14 ml de toluène. On ajoute ensuite sous agitation 0.16 g ($3,75.10^{-3}$ mole) de chlorure de lithium, 70 mg ($6.10^{-5}$ mole) de tétrakis(triphénylphosphine) palladium et 3 ml ($3.10^{-3}$ mole) d'une solution 1M de carbonate de sodium. Le mélange réactionnel est ensuite porté à reflux sous agitation pendant 14 heures puis les solvants sont chassés sous pression réduite. Le solide résiduel est purifié par chromatographie sur gel de silice en éluant à l'aide d'un mélange éther de pétrole/acétate d'éthyle (9/1 ; v/v). On obtient ainsi 225 mg du produit attendu sous forme d'un solide jaune pâle (rendement = 55 %).
RMN $^1H$ (300MHz, DMSO) $\delta$ : 11.5 (s, 1H) ; 7.73 (m, 1H) ; 7.65 (s, 1H) ; 7.61 (s, 1H) ; 7.50 (d, J = 8.5 Hz, 1H) ; 7.35 (d, J = 8.4 Hz, 1H) ; 7.12 (d, J = 8.4 Hz, 1H) ; 3.56 (s, 3H) ; 2.84 (m, 2H) ; 2.37 (m, 2H) ; 1.83 (m, 2H).

**Exemple 20**

**Acide 5-chloro-2-(4-chloro-3-fluorophényl)-1*H*-indole-3-butanoïque**

En opérant de façon analogue à l'exemple 6, au départ de l'ester obtenu selon la préparation XXV, on obtient le produit attendu sous forme d'un solide jaune pâle (rendement = 53 %).
F = 182-186 °C

**PREPARATION XXVI**

**Acide 5-chloro-2-(3,4-diméthylphényl)-1*H*-indole-3-butanoïque, méthyl ester**

En opérant de façon analogue à la préparation XXV, au départ d'acide 3,4-diméthylphénylboronique, on obtient le produit attendu sous forme d'un solide jaune mal cristallisé (rendement = 77 %).
RMN $^1H$ (300MHz, $CDCl_3$) $\delta$ : 8.02 (s, 1H) ; 7.57 (d, J = 2.0 Hz, 1H) ; 7.32-7.20 (m, 4H) ; 7.13 (dd, J = 2.0 Hz, J = 8.5 Hz, 1H) ; 3.63 (s, 3H) ; 2.88 (t, J = 7.3 Hz, 2H) ; 2.34 (m, 8H) ; 2.01 (quint, J = 7.3 Hz, 2H).

## Exemple 21

**Acide 5-chloro-2-(3,4-diméthylphényl)-1*H*-indole-3-butanoïque**

En opérant de façon analogue à l'exemple 6, au départ de l'ester obtenu selon la préparation XXVI, on obtient le produit attendu sous forme d'un solide jaune (rendement = 90 %).
F = 130-134 °C

## PREPARATION XXVII

**Acide 5-chloro-2-(3-chloro-4-fluorophényl)-1*H*-indole-3-butanoïque, méthyl ester**

En opérant de façon analogue à la préparation XXV, au départ d'acide 3-chloro-4-fluorophénylboronique, on obtient le produit attendu sous forme d'un solide orange (rendement = 31 %).
F = 90-95 °C

## Exemple 22

**Acide 5-chloro-2-(3-chloro-4-fluorophényl)-1*H*-indole-3-butanoïque**

En opérant de façon analogue à l'exemple 6, au départ de l'ester obtenu selon la préparation XXVII, on obtient le produit attendu sous forme d'un solide blanc cassé (rendement = 77 %).
F = 171-175 °C

## PREPARATION XXVIII

**Acide 2-bromo-5-chloro-1*H*-indole-3-butanoïque, éthyl ester**

En opérant de façon analogue à la préparation XXIV, au départ de l'ester éthylique de l'acide 5-chloro-1*H*-indole-3-butanoïque, on obtient le produit attendu sous forme d'un solide beige (rendement = 94 %).
F = 108-110 °C

## PREPARATION XXIX

**Acide 5-chloro-2-(3-chlorophényl)-1*H*-indole-3-butanoïque, éthyl ester**

En opérant de façon analogue à la préparation XXV, au départ du composé obtenu selon la préparation XXVIII et d'acide 4-chlorophénylboronique, on obtient le produit attendu sous forme d'un solide jaune (rendement = 83 %).
F = 79-81 °C

## Exemple 23

**Acide 5-chloro-2-(3-chlorophényl)-1*H*-indole-3-butanoïque**

En opérant de façon analogue à l'exemple 6, au départ de l'ester obtenu selon la préparation XXIX, on obtient le produit attendu sous forme d'un solide beige (rendement = 64 %).
F = 115-116 °C

## PREPARATION XXX

**Acide 5-chloro-2-[4-(trifluorométhyl)phényl]-1*H*-indole-3-butanoïque, méthyl ester**

En opérant de façon analogue à la préparation XXV, au départ d'acide 4-(trifluorométhyl)phénylboronique, on obtient le produit attendu sous forme d'une pâte marron (rendement = 29 %).
RMN $^1H$ (300MHz, DMSO) $\delta$ : 11.57 (s, 1H) ; 7.86 (s, 4H) ; 7.68 (s, 1H) ; 7.40 (d, J = 8.5 Hz, 1H) ; 7.14 (d, J = 8.5 Hz, 1H) ; 4.02 (q, J = 7.0 Hz, 2H) ; 2.87 (t, J = 6.6 Hz, 2H) ; 2.35 (t, J = 6.6 Hz, 2H) ; 1.86 (quint, J = 6.6 Hz, 2H) ; 1.15 (t, J = 7.0 Hz, 3H).

**Exemple 24**

**Acide 5-chloro-2-[4-(trifluorométhyl)phényl]-1*H*-indole-3-butanoïque**

En opérant de façon analogue à l'exemple 6, au départ de l'ester obtenu selon la préparation XXX, on obtient le produit attendu sous forme d'un solide blanc (rendement = 65 %).
F = 132-134 °C

**PREPARATION XXXI**

**Acide 5-chloro-2-(4-fluoro-3-méthylphényl)-1*H*-indole-3-butanoïque, éthyl ester**

En opérant de façon analogue à la préparation XXIX, au départ d'acide 4-fluoro-3-méthylphénylboronique, on obtient le produit attendu sous forme d'un solide jaune pâle (rendement = 87 %).
F = 118-120 °C

**Exemple 25**

**Acide 5-chloro-2-(4-fluoro-3-méthylphényl)-1*H*-indole-3-butanoïque**

En opérant de façon analogue à l'exemple 6, au départ de l'ester obtenu selon la préparation XXXI, on obtient le produit attendu sous forme d'un solide jaune pâle (rendement = 88 %).
F = 154-155 °C

**PREPARATION XXXII**

**Acide 5-chloro-2-(4-fluoro-3-méthylphényl)-1*H*-indole-3-butanoïque, méthyl ester**

En opérant de façon analogue à la préparation XXV, au départ d'acide 4-chloro-3-méthylphénylboronique, on obtient le produit attendu sous forme d'une pâte jaune (rendement = 88 %).
RMN $^1H$ (300MHz, $CDCl_3$) $\delta$ : 8.01 (s, 1H) ; 7.57 (d, J = 2.2 Hz, 1H) ; 7.43 (d, J = 8.5 Hz, 1H) ; 7.40 (d, J = 1.5 Hz, 1H) ; 7.30 (dd, J = 2.2 Hz, J = 8.5 Hz, 1H) ; 7.23 (d, J = 8.5 Hz, 1H) ; 7.17 (dd, J =1.5 Hz, J = 8.5 Hz, 1H) ; 3.63 (s, 3H) ; 2.86 (t, J = 6.7 Hz, 2H) ; 2.46 (s, 3H) ; 2.34 (t, J = 6.7 Hz, 2H) ; 2.00 (quint, J = 6.7 Hz, 2H).

**Exemple 26**

**Acide 5-chloro-2-(4-chloro-3-méthylphényl)-1*H*-indole-3-butanoïque**

En opérant de façon analogue à l'exemple 6, au départ de l'ester obtenu selon la préparation XXXII, on obtient le produit attendu sous forme d'un solide jaune (rendement = 86 %).
F = 173-174 °C

**PREPARATION XXXIII**

**Acide 5-chloro-2-(4-nitrophényl)-1*H*-indole-3-butanoïque, éthyl ester**

On prépare un mélange de 200 mg ($0,58.10^{-3}$ mole) d'ester éthylique de l'acide 2-bromo-5-chloro-1*H*-indole-3-butanoïque, 498 mg ($1,74.10^{-3}$ mole) de triméthyl(4-nitrophényl)étain, 140 mg ($0,46.10^{-3}$ mole) de triphénylarsine, 108 mg ($0,12.10^{-3}$ mole) de tris(dibenzylidèneacétone)dipalladium et 12 ml de dioxane et on porte ce milieu réactionnel, sous agitation, à 50 °C pendant 6 jours. Après refroidissement, on ajoute 12 ml d'eau, puis on extrait le mélange avec de l'éther éthylique. La phase organique est ensuite lavée à l'eau puis séchée et concentrée sous pression réduite. Le résidu est purifié par chromatographie sur gel de silice en éluant à l'aide d'un mélange éther de pétrole/acétate d'éthyle (85/15 ; v/v). Les fractions contenant le composé attendu sont à nouveau purifiées par chromatographie en phase inverse sur silice greffée $C_{18}$ en éluant à l'aide d'un mélange acétonitrile/eau (7/3 ; v/v). On obtient ainsi 70 mg du composé attendu sous forme d'une pâte jaune (rendement = 31 %).
RMN $^1H$ (300MHz, DMSO) $\delta$ : 11.70 (s, 1H) ; 8.38 (d, J = 8.8 Hz, 2H) ; 7.93 (d, J = 8.8 Hz, 2H) ; 7.72 (d, J = 1.8 Hz, 1H) ; 7.42 (d, J = 8.8 Hz, 1H) ; 7.18 (dd, J = 1.8 Hz, J = 8.8 Hz, 1H) ; 4.05 (q, J = 7.0 Hz, 2H) ; 2.95 (m, 2H) ; 2.40 (m, 2H) ; 1.85 (m, 2H) ; 1.16 (t, J = 7.0 Hz, 3H).

**Exemple 27**

**Acide 5-chloro-2-(4-nitrophényl)-1*H*-indole-3-butanoïque**

En opérant de façon analogue à l'exemple 6, au départ de l'ester obtenu selon la préparation XXXIII, on obtient le produit attendu sous forme d'un solide jaune (rendement = 99 %).
F = 234-235 °C

**PREPARATION XXXIV**

**Acide 5-chloro-2-(3-thiényl)-1*H*-indole-3-butanoïque, méthyl ester**

En opérant de façon analogue à la préparation XXV, au départ d'acide 3-thiénylboronique. on obtient le produit attendu sous forme d'un solide jaune (rendement = 28 %).
F = 65-68 °C

**Exemple 28**

**Acide 5-chloro-2-(3-thiényl)-1*H*-indole-3-butanoïque**

En opérant de façon analogue à l'exemple 6, au départ de l'ester obtenu selon la préparation XXXIV, on obtient le produit attendu sous forme d'un solide jaune (rendement = 67 %).
F = 145-150 °C

**PREPARATION XXXV**

**Acide 5-chloro-1*H*-indole-3-propanoïque, méthyl ester**

On prépare une solution de 232 mg ($1,04.10^{-3}$ mole) d'acide 5-chloro-1*H*-indole-3-propanoïque dans 14 ml d'éthanol et on ajoute, à température ambiante, 4,4 ml ($8,8.10^{-3}$ mole) d'une solution 2M dans l'hexane de (triméthylsilyl) diazométhane. Le milieu réactionnel est agité pendant 15 mn, puis on ajoute 1 g de silice, puis on filtre le mélange. Le filtrat est concentré sous pression réduite et le résidu est purifié par chromatographie sur gel de silice en éluant à l'aide d'un mélange méthylcyclohexane/acétate d'éthyle (2/1 ; v/v). On obtient ainsi 225 mg du produit attendu sous forme d'un solide jaune (rendement = 91 %).
F = 86 °C

**PREPARATION XXXVI**

**Acide 2-bromo-5-chloro-1*H*-indole-3-propanoïque, méthyl ester**

En opérant de façon analogue à la préparation XXIV, au départ de l'ester obtenu selon la préparation XXXV, on obtient le produit attendu sous forme d'une huile marron (rendement = 77 %).
RMN $^1$H (300MHz, DMSO) δ : 11.90 (s, 1H) ; 7.58 (d, J = 1.9 Hz, 1H) ; 7.28 (d, J = 8.5 Hz) ; 7.08 (dd, J = 1.9 Hz, J = 8.5 Hz, 1H) ; 3.56 (s, 3H) ; 2.91 (t, J = 7.5 Hz, 2H) ; 2.57 (t, J = 7.5 Hz, 2H).

**PREPARATION XXXVII**

**Acide 5-chloro-2-[4-(trifluorométhyl)phényl]-1*H*-indole-3-propanoïque, méthyl ester**

En opérant de façon analogue à la préparation XXX, au départ du dérivé bromé obtenu selon la préparation XXXVI, on obtient le produit attendu sous forme d'une huile incolore (rendement = 35 %).
RMN $^1$H (300MHz, $CDCl_3$) δ : 8.11 (s, 1H) ; 7.76 (d, J = 8.2 Hz, 2H) ; 7.68 (d, J = 8.2 Hz, 2H) ; 7.62 (s, 1H) ; 7.32 (d, J = 8.6 Hz, 1H) ; 7.21 (d, J = 8.6 Hz, 1H) ; 3.65 (s, 3H) ; 3.22 (t, J = 8 Hz, 2H) ; 2.68 (t, J = 8 Hz, 2H).

**Exemple 29**

**Acide 5-chloro-2-[4-(trifluorométhyl)phényl]-1*H*-indole-3-propanoïque,**

En opérant de façon analogue à l'exemple 6, au départ de l'ester obtenu selon la préparation XXXVII, on obtient le produit attendu sous forme d'un solide beige (rendement = 76 %).
F = 218 °C

**PREPARATION XXXVIII**

**Acide 5-chloro-2-(3,5-diméthyl-4-fluorophényl)-1*H*-indolebutanoïque, méthyl ester**

En opérant de façon analogue à la préparation XXV, au départ d'acide 3,5-diméthyl-4-fluorophénylboronique, on obtient le produit attendu sous forme d'une huile épaisse jaune (rendement = 45 %).
RMN (300MHz, DMSO) δ : 11.35 (s, 1H) ; 7.60 (s, 1H) ; 7.35 (m. 3H) ; 7.10 (d, 1H) ; 3.60 (s, 3H) ; 2.82 (t, 2H) ; 2.40 (t, 2H) ; 2.35 (s, 6H) ; 1.85 (m, 2H).
L'acide 3,5-diméthyl-4-fluorophénylboronique est obtenu, avec un rendement de 22 %, selon un procédé analogue à la préparation des dérivés phénylboroniques, par action successive du n-BuLi, puis du borate d'isopropyle sur le 5-bromo-2-fluoro-1,3-diméthylbenzène
RMN (300Mhz, DMSO) δ : 7.95 (s, 1H) ; 7.50 (d, 2H) ; 6.50 (s, 1H) ; 2.20 (s, 3H).

**Exemple 30**

**Acide 5-chloro-2-(3,5-diméthyl-4-fluorophényl)-1*H*-indolebutanoïque**

En opérant de façon analogue à l'exemple 6, au départ de l'ester obtenu selon la préparation XXXVIII, on obtient le produit attendu sous forme d'un solide jaune pâle (rendement = 71 %).
F = 58 °C

**PREPARATION XXXIX**

**Acide 5-chloro-2-[4-chloro-3-(trifluorométhyl)phenyl]-1*H*-indolebutanoïque, méthyl ester**

En opérant de façon analogue à la préparation XXV, au départ d'acide 4-chloro-3-(trifluorométhyl)phénylboronique, on obtient le produit attendu sous forme d'une huile beige (rendement = 41 %).
RMN (300MHz, DMSO) δ : 11.62 (s, 1H) ; 8.05 (s, 1H) ; 7.95 (d, 1H) ; 7.85 (d, 1H) ; 7.70 (s, 1H) ; 7.40 (d, 1H) ; 7.15 (d, 1H) ; 3.60 (s, 3H) ; 2.85 (t, 2H) ; 2.35 (t, 2H) ; 1.85 (m, 2H).

**Exemple 31**

**Acide 5-chloro-2-[4-chloro-3-(trifluorométhyl)phenyl]-1*H*-indolebutanoïque**

En opérant de façon analogue à l'exemple 6, au départ de l'ester obtenu selon la préparation XXXIX, on obtient le produit attendu sous forme d'un solide blanc (rendement = 88 %).
F= 158-160 °C
Le tableau I résume les formules des composés précédemment décrits.

TABLEAU I

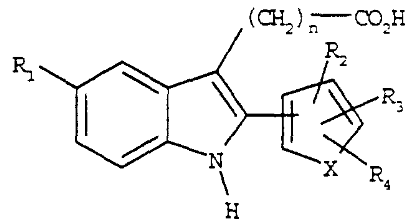

| Exemple | X | $R_1$ | n | $R_2$ | $R_3$ | $R_4$ |
|---|---|---|---|---|---|---|
| 1 | -C=C- | Br | 3 | H | H | H |
| 2 | -C=C- | Cl | 2 | H | H | H |
| 3 | -C=C- | Cl | 3 | H | H | H |
| 4 | -C=C- | F | 3 | H | H | H |
| 5 | -C=C- | $CF_3$ | 3 | H | H | H |
| 6 | -C=C- | Cl | 2 | 4-F | H | H |
| 7 | -C=C- | Cl | 2 | 4-Cl | H | H |
| 8 | -C=C- | Cl | 2 | 4-$CH_3$ | H | H |
| 9 | -C=C- | Cl | 3 | 4-Cl | H | H |
| 10 | -C=C- | Cl | 3 | 3-Cl | 4-Cl | H |
| 11 | -C=C- | $CH_3$ | 3 | H | H | H |
| 12 | -C=C- | Cl | 3 | 4-F | H | H |
| 13 | -C=C- | Cl | 3 | * | * | H |
| 14 | -C=C- | $NO_2$ | 3 | H | H | H |
| 15** | -C=C- | Cl | 3 | 4-F | H | H |
| 16 | -C=C- | Cl | 2 | 3-Cl | 4-Cl | H |
| 17 | -C=C- | Cl | 3 | 4-Br | H | H |
| 18 | -C=C- | Cl | 3 | 4-CN | H | H |
| 19 | -C=C- | Cl | 3 | 3-F | 4-F | H |
| 20 | -C=C- | Cl | 3 | 3-F | 4-Cl | H |
| 21 | -C=C- | Cl | 3 | 3-$CH_3$ | 4-$CH_3$ | H |
| 22 | -C=C- | Cl | 3 | 3-Cl | 4-F | H |
| 23 | -C=C- | Cl | 3 | 3-Cl | H | H |
| 24 | -C=C- | Cl | 3 | 4-$CF_3$ | H | H |
| 25 | -C=C- | Cl | 3 | 3-$CH_3$ | 4-F | H |
| 26 | -C=C- | Cl | 3 | 3-$CH_3$ | 4-Cl | H |
| 27 | -C=C- | Cl | 3 | 4-$NO_2$ | H | H |
| 28 | S | Cl | 3 | H | H | H |
| 29 | -C=C- | Cl | 2 | 4-$CF_3$ | H | H |
| 30 | -C=C- | Cl | 3 | 3-$CH_3$ | 4-F | 5-$CH_3$ |
| 31 | -C=C- | Cl | 3 | 3-$CF_3$ | 4-Cl | H |

* $R_2$ et $R_3$ forment un groupe 2-naphtyle avec le phényl

** sel de sodium de l'exemple 12

## Activité biologique

Les effets inhibiteurs des composés décrits dans la présente invention sur les chimiokines IL-8 et Gro-alpha ont été déterminés par les tests *in vitro* suivants:

### A)Test de liaison aux récepteurs de l'IL-8

L'IL-8 humaine marquée à l'iode 125 ([$^{125}$I]-IL-8) a été obtenue de NEN (Les Ulis) et possède une activité spécifique de 2.200 Ci/mmol. Le récepteur CXCR2 humain recombinant a été exprimé dans des cellules HEK 293 (ATCC. CRL-1573) K-562 (ATCC. CCL-243) ou THP-1 (ATCC, TIB-202). Les cellules HEK 293 sont maintenues en culture dans du milieu DMEM (GIBCO) contenant 4.5 g/l de glucose, 10 % de sérum de veau foetal, 1% de Glutamax, 1% d'acides aminés non essentiels, 1 mM de sodium pyruvate, 100 UI/ml de pénicilline et 100 μg/ml de streptomycine. Les cellules K-562 et THP-1 sont maintenues en culture dans du milieu RPMI1640 (GIBCO) contenant 10 % de sérum de veau foetal. 1% d'acides aminés non essentiels. 1 mM de sodium pyruvate, 100 UI/ml de pénicilline et 100 μg/ml de streptomycine. Les cellules sont utilisées lorsque les cultures ont atteint 80 % de confluence.

Les membranes sont préparées selon le protocole précédemment décrit (Bastian *et al*, *Br. J. Pharmacol.* 1997, **122**, 393-399) excepté le tampon d'homogénéisation qui a été remplacé par une solution saline tamponnée à pH 8,0 contenant 20 mM Tris. 1,2 mM $MgSO_4$. 0.1 mM EDTA et 25 mM NaCl. Les expériences de compétition sont réalisées dans des plaques 96 puits de 1 ml. à température ambiante, sous un volume final de 0.25 ml. Les membranes diluées dans une solution de 20 mM Bis-Trispropane et de 0,4 mM Tris-HCl tamponnée à pH 8.0 contenant 1,2 mM de $MgSO_4$. 0.1 mM EDTA 25 mM NaCl et 0.03 % CHAPS sont incubées avec des concentrations décroissantes du composé à tester (de 100 μM à 0.01 nM) et 150 pM de [$^{125}$I]-IL-8. La liaison non-spécifique est déterminée en présence de 300 nM d'IL-8 non marquée. Après 60 min d'incubation à température ambiante. la réaction est stoppée par filtration rapide sous vide sur filtre Whatman GF/C préalablement incubé pendant 1 heure à + 4 °C dans une solution de polyéthylènimine 1 % (poids/volume) et SAB 0.5 % (poids/volume). Les filtres sont lavés avec une solution contenant 25 mM de NaCl, 1 mM de $MgSO_4$, 0.5 mM d'EDTA et 10 mM de Tris-HCl tamponnée à pH 7,4. La radioactivité retenue sur les filtres est mesurée dans un compteur gamma.

Les affinités des composés décrits dans la présente invention ont été aussi déterminées par un test de liaison sur cellules entières. Les cellules THP-1 ou K-562 transfectées sont mises en suspension dans le tampon de test de liaison (PBS sans calcium ni magnésium contenant 0,5 % de SAB (poids/volume), pH 7,4) à raison de 2,5 x $10^6$ cellules/ml. Les expériences de compétition sont réalisées dans des plaques 96 puits de 1 ml dans un volume final de 0,25 ml. 0,5 x $10^6$ cellules sont incubées avec des concentrations décroissantes du composé à tester (100 μM à 0,01 nM) et 150 pM de [$^{125}$I]-IL-8. La liaison non-spécifique est déterminée en présence de 300 nM de chimiokine non radiomarquée. Après 90 min d'incubation à + 4 °C, la réaction est stoppée par filtration rapide sous vide sur filtre Whatman GF/C préalablement incubé pendant 1 h dans une solution de polyéthylènimine 3 % (poids/volume). Les filtres sont lavés avec une solution de PBS à pH 7,4 contenant 0,5 M de NaCl. La radioactivité contenue dans les filtres est mesurée dans un compteur gamma.

Les composés de formule I décrits dans la présente invention testés à la concentration de 10 μM inhibent de 95 % au moins la liaison de la [$^{125}$I]-IL-8 sur le récepteur CXCR2.

### B) Mesure des flux calciques

Les effets des composés de la présente invention ont été évalués sur les flux calciques induits par l'IL-8 ou le Gro-alpha.

Des cellules THP-1 exprimant les récepteurs CXCR2 recombinants, des cellules U937 différentiées avec du DMSO (Diméthyl sulfoxyde) à 1 % (volume/volume) ou des cellules Eol3 sont incubées en présence d'un indicateur fluorescent, le Fura-2 AM, à la concentration de 5 μM pendant 1 h à 37°C. Après cette période de charge, les cellules sont lavées, et mises en suspension à la concentration de 1 x $10^6$ cellules/ml dans une solution saline contenant : 136 mM NaCl, 4,7 nM KCl, 1,2 mM $MgSO_4$, 1,6 mM $CaCl_2$, 1,2 mM $KH_2PO_4$, 11 mM glucose, 5 mM HEPES, pH 7,4. La suspension cellulaire (2 ml) est placée dans une cuve en quartz et l'intensité de fluorescence à 510 nm est mesurée sur un spectrofluorimètre de type LS50B (Perkin-Elmer) après des excitations alternativement à 340 nm et 380 nm. Le rapport des intensités de fluorescence après excitation à 340 nm et 380 nm est déterminé et la concentration calcique intracellulaire est calculée suivant la formule :

$$[Ca^{2+}]i = K_d \frac{(R\text{-}Rmin)}{(Rmax\text{-}R)} (Sf2/Sb2)$$

dans laquelle :

$K_d$ représente la constante d'affinité du complexe Fura-2 et calcium, Rmax est l'intensité de fluorescence maximale déterminée après addition de 1 μM du ionophore Bromo-A23187, Rmin est le rapport minimal déterminé après addition de 10 mM d'EGTA consécutif à l'addition d'ionophore et Sf2/Sb2 est le rapport des valeurs de fluorescence sous excitation à 380 nm déterminé aux Rmin et Rmax, respectivement.

Après une période de stabilisation de 1 min, pendant laquelle la concentration calcique intracellulaire basale est déterminée, le composé à tester ou le véhicule contrôle est ajouté aux cellules. Après une période d'incubation de 2 min pendant laquelle la concentration de calcium est mesurée, les cellules sont stimulées avec les différents agonistes (IL-8 ou Gro-alpha). La concentration calcique est mesurée pendant 2 min.

Les composés de formule I décrits dans la présente invention inhibent la libération de calcium induite par l'IL-8 ou le Gro-alpha.

L'activité des composés selon l'invention, mise en évidence au cours des tests biologiques, est significative d'une action antagoniste de l'IL-8 et permet d'envisager leur utilisation en thérapeutique.

Selon l'invention, on préconise l'utilisation des composés de formule I en tant que principes actifs de médicaments destinés à un traitement préventif ou curatif chez les mammifères, notamment chez l'homme, vis à vis des maladies en relation avec une mise en jeu de l'IL-8 et/ou de chimiokines de la même famille, et qui sont généralement caractérisées par une invasion massive de neutrophiles.

Parmi les maladies qui peuvent être traitées en administrant une quantité thérapeutiquement suffisante d'au moins l'un des composés de formule I, on peut citer la polyarthrite rhumatoïde, le psoriasis ou les dermatites atypiques, les maladies associées à une angiogénèse pathologique (comme le cancer), la prolifération des cellules tumorales et la formation de métastases (dans le cas du mélanome par exemple), l'asthme, l'obstruction chronique des poumons, le syndrome de détresse respiratoire aiguë, l'inflammation du colon, la maladie de Crohn, la colite ulcérative, l'ulcère gastrique, le choc septique, le choc endotoxinique, la septicémie à bactéries gram (-), le syndrome de choc toxique, l'ischémie cérébrale, les phénomènes d'ischémie/reperfusion cardiaques ou rénaux, les glomérulo-néphrites, la thrombose, l'athérome, la maladie d'Alzheimer, les réactions du greffon contre l'hôte ou les rejets d'allogreffes.

Les composés de formule I doivent être administrés en quantité suffisante pour antagoniser l'IL-8 en se fixant de façon compétitive sur les récepteurs. La dose de principe actif dépend du mode d'administration et du type de pathologie et est généralement comprise entre 0,01 et 10 mg/kg. Les composés de formule I peuvent également être associés à un autre principe actif.

Dans le cadre de leur utilisation thérapeutique, les composés de formule I seront généralement administrés sous des formes variées, en association avec les excipients couramment utilisés.

La formulation utilisée pourra être une forme orale, telle que par exemple des gélules, des comprimés contenant le principe actif solide sous une forme pulvérisée ou micronisée, un sirop ou une solution contenant le principe actif en solution, en suspension, en émulsion ou en microémulsion.

La formulation peut également se présenter sous une forme administrable pour un usage topique, par exemple une crème ou une lotion ou un dispositif transdermique tel qu'un patch adhésif. On peut également formuler le principe actif pour un mode d'administration par injection sous-cutanée, intramusculaire ou intraveineuse.

## Revendications

1. Composé dérivé de l'indole, **caractérisé en ce qu'**il est choisi parmi l'ensemble constitué par :

i) les produits de formule :

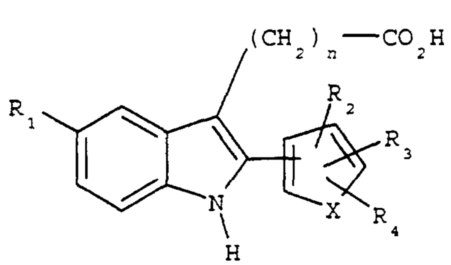

(I)

dans laquelle

X représente une double liaison -C=C- ou un atome de soufre,

$R_1$ représente un halogène, un groupe nitro, un groupe trifluorométhyle ou un groupe alkyle en $C_1$-$C_3$, $R_2$, $R_3$ et $R_4$ représentent chacun indépendamment un atome d'hydrogène, un halogène, un groupe alkyle en $C_1$-$C_3$, un groupe nitro, un groupe trifluorométhyle ou un groupe cyano, ou $R_2$ et $R_3$ forment ensemble, avec le noyau aromatique auquel ils sont rattachés, un cycle aromatique condensé, et n est égal à 2 ou 3 et

ii) les esters et les sels d'addition avec une base minérale ou organique des composés de formule I.

**2.** Composé dérivé de l'indole de formule (I) selon la revendication 1, dans laquelle X représente une double liaison -C=C-, **caractérisé en ce qu'**il est choisi parmi l'ensemble constitué par :

i) les produits de formule :

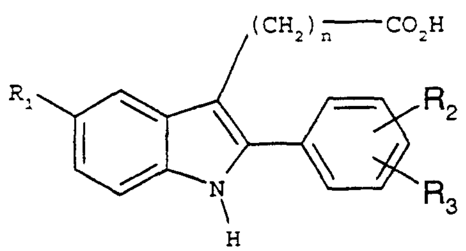

(Ia)

dans laquelle $R_1$ représente un halogène, un groupe nitro, un groupe trifluorométhyle ou un groupe alkyle en $C_1$-$C_3$,
$R_2$ et $R_3$ représentent chacun indépendamment un atome d'hydrogène, un halogène, un groupe alkyle en $C_1$-$C_3$, ou forment ensemble, avec le noyau phényle auquel ils sont attachés, un cycle aromatique condensé, et n est égal à 2 ou 3 et
ii) les esters et les sels d'addition avec une base minérale ou organique des composés de formule I.

**3.** Composé selon l'une des revendications 1 ou 2, **caractérisé en ce que** $R_2$ représente un atome d'hydrogène, un atome de chlore ou un atome de fluor ou un groupe méthyle, $R_3$ représente un atome de chlore ou un atome de fluor et $R_4$ représente un atome d'hydrogène.

**4.** Composé selon la revendication 1, **caractérisé en ce que** X représente une double liaison -C=C- et $R_2$ et $R_3$ forment ensemble avec le noyau phényle auquel ils sont rattachés, un groupe naphtyle.

**5.** Composé selon la revendication 2, **caractérisé en ce que** $R_2$ et $R_3$ forment ensemble avec le noyau phényle auquel ils sont rattachés, un groupe naphtyle.

**6.** Composé selon l'une des revendications 1 à 5, **caractérisé en ce que** $R_1$ représente un atome de chlore.

**7.** Composition thérapeutique **caractérisée en ce qu'**elle renferme, en association avec au moins un excipient physiologiquement acceptable, au moins un composé dérivé de l'indole de formule (I) selon l'une des revendications 1 à 6 ou l'un de ses sels.

**8.** Utilisation d'un composé dérivé de l'indole de formule (I) selon l'une des revendications 1 à 6 ou de l'un de ses sels d'addition, pour la préparation d'un médicament destiné au traitement ou à la prévention des maladies mettant en cause une surexpression de l'IL-8 et/ou de chimiokines de la même famille.

### Patentansprüche

**1.** Von Indol abgeleitete Verbindung, **dadurch gekennzeichnet, daß** sie ausgewählt ist aus der Gruppe der:

i) Produkte der Formel:

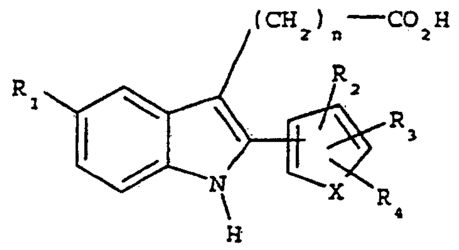

(I)

wobei

X eine Doppelbindung -C=C- oder ein Schwefelatom darstellt,
$R_1$ ein Halogenatom, eine Nitrogruppe, eine Trifluormethylgruppe oder einen $C_1$-$C_3$-Alkylrest darstellt,
$R_2$, $R_3$ und $R_4$ jeweils unabhängig ein Wasserstoffatom, ein Halogenatom, einen $C_1$-$C_3$-Alkylrest, eine Nitrogruppe, eine Trifluormethylgruppe oder eine Cyanogruppe darstellen oder $R_2$ und $R_3$ zusammen mit dem aromatischen Ring, an den sie gebunden sind, einen kondensierten aromatischen Ring bilden,
und n gleich 2 oder 3 ist, und
ii) Ester und Additionssalze der Verbindungen der Formel I mit einer anorganischen oder organischen Base.

2. Von Indol abgeleitete Verbindung der Formel (I) nach Anspruch 1, wobei X eine Doppelbindung -C=C- darstellt, **dadurch gekennzeichnet, daß** sie ausgewählt ist aus der Gruppe der:

i) Produkte der Formel:

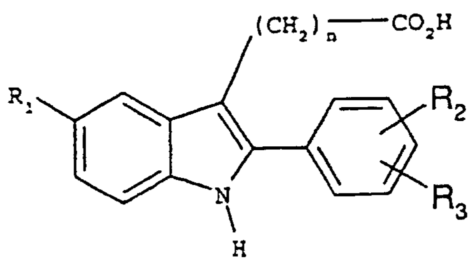

(Ia)

wobei $R_1$ ein Halogenatom, eine Nitrogruppe, eine Trifluormethylgruppe oder einen $C_1$-$C_3$-Alkylrest darstellt,
$R_2$ und $R_3$ jeweils unabhängig ein Wasserstoffatom, ein Halogenatom, einen $C_1$-$C_3$-Alkylrest darstellen oder zusammen mit dem Phenylring, an den sie gebunden sind, einen kondensierten aromatischen Ring bilden,
und n gleich 2 oder 3 ist, und
ii) Ester und Additionssalze der Verbindungen der Formel I mit einer anorganischen oder organischen Base.

3. Verbindung nach einem der Ansprüche 1 oder 2, **dadurch gekennzeichnet, daß** $R_2$ ein Wasserstoffatom, ein Chloratom oder ein Fluoratom oder eine Methylgruppe darstellt, $R_3$ ein Chloratom oder ein Fluoratom darstellt und $R_4$ ein Wasserstoffatom darstellt.

4. Verbindung nach Anspruch 1, **dadurch gekennzeichnet, daß** X eine Doppelbindung -C=C- darstellt und $R_2$ und $R_3$ zusammen mit dem Phenylring, an den sie gebunden sind, eine Naphthylgruppe bilden.

5. Verbindung nach Anspruch 2, **dadurch gekennzeichnet, daß** $R_2$ und $R_3$ zusammen mit dem Phenylring, an den sie gebunden sind, eine Naphthylgruppe bilden.

6. Verbindung nach einem der Ansprüche 1 bis 5, **dadurch gekennzeichnet, daß** $R_1$ ein Chloratom darstellt.

7. Therapeutisches Mittel, **dadurch gekennzeichnet, daß** es in Verbindung mit mindestens einem physiologisch verträglichen Exzipienten mindestens eine von Indol abgeleitete Verbindung der Formel (I) nach einem der Ansprüche 1 bis 6 oder eines ihrer Salze beinhaltet.

**8.** Verwendung einer von Indol abgeleiteten Verbindung der Formel (I) nach einem der Ansprüche 1 bis 6 oder eines ihrer Additionssalze zur Herstellung eines Medikaments zur Behandlung oder Prävention von Krankheiten, die durch eine Überexpression von IL-8 und/oder Chemokinen der gleichen Familie hervorgerufen werden.

## Claims

**1.** A compound derived from indole, **characterised in that** it is selected from the group consisting of:

i) the products of the formula

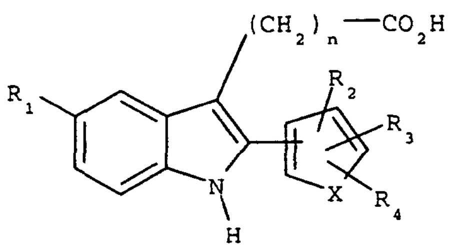

(I)

in which:

X is a double bond -C=C- or a sulfur atom;
$R_1$ is a halogen, a nitro group, a trifluoromethyl group or a $C_1$-$C_3$ alkyl group;
$R_2$, $R_3$ and $R_4$ are each independently a hydrogen atom, a halogen, a $C_1$-$C_3$ alkyl group, a nitro group, a trifluoromethyl group or a cyano group, or $R_2$ and $R_3$ form a fused aromatic ring together with the aromatic ring to which they are attached; and
n is equal to 2 or 3; and

ii) esters of the compounds of formula I and addition salts of said compounds with a mineral or organic base.

**2.** A compound derived from indole of formula (**I**) according to claim 1 in which X is a double bond -C=C- , **characterised in that** it is selected from the group consisting of:

i) the products of the formula

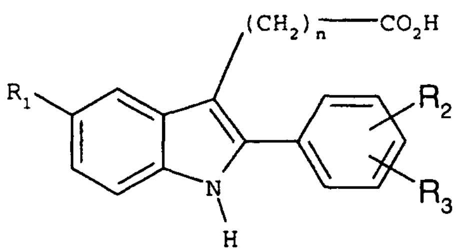

(Ia)

in which:

$R_1$ is a halogen, a nitro group, a trifluoromethyl group or a $C_1$-$C_3$ alkyl group;
$R_2$ and $R_3$ are each independently a hydrogen atom, a halogen or a $C_1$-$C_3$ alkyl group, or they form a fused aromatic ring together with the phenyl ring to which they are attached; and
n is equal to 2 or 3; and

ii) esters of the compounds of formula I and addition salts of said compounds with a mineral or organic base.

**3.** A compound according to one of claims 1 or 2, **characterised in that** $R_2$ is a hydrogen atom, a chlorine atom, a fluorine atom or a methyl group, $R_3$ is a chlorine atom or a fluorine atom and $R_4$ is a hydrogen atom.

**4.** A compound according to claim 1, **characterised in that** X is a double bond -C=C- and $R_2$ and $R_3$ form a naphthyl group together with the phenyl ring to which they are attached.

**5.** A compound according to claim 2, **characterised in that** $R_2$ and $R_3$ form a naphthyl group together with the phenyl ring to which they are attached.

**6.** A compound according to one of claims 1 to 5, **characterised in that** $R_1$ is a chlorine atom.

**7.** A therapeutic composition **characterised in that** it contains, in association with at least one physiologically acceptable excipient, at least one compound derived from indole of formula (I) according to one of claims 1 to 6, or one of its salts.

**8.** Use of a compound derived from indole of formula (I) according to one of claims 1 to 6, or one of its addition salts, for the preparation of a drug for the treatment or prevention of diseases involving an overexpression of IL-8 and/or chemokines of the same family.